# EUROPÄISCHE PATENTSCHRIFT

(11) **EP 1 019 518 B1**
(45) Veröffentlichungstag und Bekanntmachung des Hinweises auf die Patenterteilung: **04.01.2006**
(21) Anmeldenummer: 99926413.8
(22) Anmeldetag: 25.05.1999
(51) Int. Cl.: C12N 15/85, A61K 48/00

(54) **SELBSTDELETIERENDE VEKTOREN FÜR DIE KREBSTHERAPIE**
SELF-DELETING VECTORS FOR CANCER THERAPY
VECTEURS D'AUTODETECTION UTILISES DANS LE TRAITEMENT DE CANCERS

(30) Priorität: 30.07.1998 DE 19834430
(43) Veröffentlichungstag der Anmeldung: 19.07.2000
(73) Patentinhaber: FrankGen Biotechnologie AG, 36391 Sinntal (DE)
(72) Erfinder: VON MELCHNER, Harald,Prof.Dr. Universitätsklinikum, 60596 Frankfurt am Main (DE); EBENSPERGER, Christoph, Universitätskli. Frankfurt, 60596 Frankfurt am Main (DE); ANDRE, Thomas, Universitätsklinik. Frankfurt, 60596 Frankfurt am Main (DE)
(74) Vertreter: Helbing, Jörg
(86) Internationale Anmeldenummer: PCT/EP1999/003607
(87) Internationale Veröffentlichungsnummer: WO 2000/006758

(56) Entgegenhaltungen:
- WO-A-97/07223
- KERN S E ET AL: "ONCOGENIC FORMS OF P53 INHIBITS P53-REGULATED GENE EXPRESSION" SCIENCE,US,AMERICAN ASSOCIATION FOR THE ADVANCEMENT OF SCIENCE,, Bd. 256, Seite 827-830 XP000619184 ISSN: 0036-8075 in der Anmeldung erwähnt
- ROSSI F ET AL: "EXCISION OF ETS BY AN INDUCIBLE SITE-SPECIFIC RECOMBINASE CAUSES DIFFERENTIATION OF MYB-ETS-TRANSFORMED HEMATOPOIETIC PROGENITORS" CURRENT BIOLOGY,GB,CURRENT SCIENCE,, Bd. 6, Nr. 7, Seite 866-872 XP000617923 ISSN: 0960-9822
- MASSUDA, E. ET AL.: "Regulated expression of the diphteria toxin A chain by a tumor-specific chimeric transcription factor results in selective toxicity for alveolar rhabdomyosarcoma cells" PROCEEDINGS OF THE NATIONAL ACADEMY OF SCIENCES, Bd. 94, 1997, Seiten 14701-14706, XP002126740
- RING, C.J.A. ET AL.: "Suicide gene expression induced in tumour cells transduced with recombinant adenoviral, retroviral and plasmid vectors containing the ERBB2 promoter" GENE THERAPY, Bd. 3, 1996, Seiten 1094-1103, XP002126741
- OZAKI, K. ET AL.: "Use of von Willebrand factor promoter to transduce suicidal gene to human endothelial cells, HUVEC" HUMAN GENE THERAPY, Bd. 7, 20. August 1996 (1996-08-20), Seiten 1483-1490, XP002126742
- MILLER ET AL: "PROGRESS IN TRANSCRIPTIONALLY TARGETED AND REGULATABLE VECTORS FOR GENETIC THERAPY" HUMAN GENE THERAPY, Bd. 8, 1. Mai 1997 (1997-05-01), Seite 803-815 XP002088320 ISSN: 1043-0342

## Beschreibung

Die vorliegende Erfindung betrifft einen rekombinanten Vektor, eine den erfindungsgemäßen Vektor enthaltende pharmazeutische Zusammensetzung, die Verwendung des erfindungsgemäßen Vektors zur Herstellung eines Arzneimittels für die Krebstherapie und transduzierte eukaryontische Zellen.

Die Therapie von benignen oder malignen Tumoren erfolgt bisher überwiegend entweder invasiv, d.h. durch chirurgische Entfernung des Tumors, oder konservativ, z.B. durch Verabreichung von Cytostatika oder Bestrahlen der betroffenen Organe, oder durch eine Kombination der erwähnten Methoden. Aufgrund sich stetig verbessernder Operationstechniken und einer enormen Entwicklung auf dem Gebiet der Cytostatika werden mit diesen Therapiemöglichkeiten bereits beachtliche Erfolge erzielt.

Dennoch sind die Erfolgsaussichten bei der Behandlung eines Tumors durch die erwähnten therapeutischen Ansätze sehr unterschiedlich und unsicher. Darüber hinaus ist jeder der drei Ansätze mit gravierenden Nachteilen verbunden. So bedeutet die operative Entfernung eines Tumors und gegebenenfalls dessen Umschneidung im gesunden Gewebe eine erhebliche Beeinträchtigung des Patienten aufgrund des Eingriffs selber. Die Behandlung mit Cytostatika sowie die Bestrahlung von Tumoren läßt sich nur in Ausnahmefällen auf die eigentlichen Zielzellen beschränken, so daß es nahezu unumgänglich ist, gesunde Zellen bzw. gesundes Gewebe mit zu behandeln. Eine Behandlung mit Cytostatika bedeutet die Inhibition von Mitosen, was z.B. als unangenehme Begleiterscheinung zum Haarausfall führt. Sowohl die cytostatische Behandlung als auch eine Strahlentherapie können bei Patienten im gebär- bzw. zeugungsfähigen Alter den Verzicht auf Kinder bedeuten.

Aufgrund der-zuvor erwähnten Beeinträchtigungen ist bereits versucht worden, Therapeutika, insbesondere Cytostatika, zielgerichtet zu Tumorzellen zu transportieren und sie von ihnen internalisieren zu lassen. Ein Beispiel dafür ist der Versuch, Cytostatika an Antikörper zu koppeln, die an tumorzellspezifische Antigene binden. Trotz der theoretischen Attraktivität dieses Vorschlags ist er jedoch mit erheblichen Schwierigkeiten, z.B. bei der Beschaffung ausreichender Mengen von Antikörpern, sowie mit ebenfalls erheblichen Risiken, wie z.B. einer immunologischen Reaktion auf das zugeführte Fremdprotein, verbunden.

Ein weiterer Ansatz beruht auf der Kenntnis der Rolle von Transkriptionsfaktoren bei der Tumorentstehung. Es ist allgemein bekannt, daß einige nukleäre Transkriptionsfaktoren der Überwachung der Integrität des zellulären Genomes dienen Wird die genomische DNA beschädigt, so induzieren diese Transkriptionsfaktoren entweder einen zur Reparatur notwendigen Zellzyklusarrest oder, bei irreparablen Schäden, die Apoptose. Dadurch besitzen diese Transkriptionsfaktoren eine wichtige Tumorsuppressorfunktion. Eine große Anzahl von Arbeiten der letzten Jahre hat sich mit dem Transkriptionsfaktor p53 beschäftigt, insbesondere mit dem Zusammenhang von gestörter p53-Funktion mit der Entstehung von Krebs (Levine, 1997). Der Phänotyp von p53^{-/-}-Mäusen, erzeugt mittels "Gene-Targeting", belegt diesen Zusammenhang: Das Fehlen des Proteins führt zum verstärkten Auftreten spontaner Tumoren (Donehower et al., 1992). Darüber hinaus konnte mit Hilfe dieser Mäuse gezeigt werden, daß p53 bei der Induktion von Apoptose eine Schlüsselrolle spielt (Lowe et al., 1994).

Bei herkömlichen Tumortherapien, wie einer Bestrahlung oder Chemotherapie, spielt gerade diese p53-vermittelte Apoptose-Induktion eine wichtige Rolle (Lowe et al., 1993). Aufgrund der damit verbundenen Resistenz gegenüber Cytostatika oder Bestrahlungstherapie haben Krebsarten mit mutiertem p53 (p53^{mut}) meistens eine schlechte Prognose.
Beim Menschen treten Mutationen von oder Deletionen in p53 in 50-80% aller Krebsarten auf (Levine et al., 1991). Sie betreffen immer die DNA-Birrdungsdomäne und haben den Verlust der p53-Transaktivatorfunktion zur Folge.

Der genetische Unterschied zwischen p53-Molekülen in normalen und transformierten Zellen wurde kürzlich zur selektiven Eliminierung von Tumorzellen ausgenutzt. Eine Adenovirus-Mutante, die sich nur in p53-defizienten Zellen vermehren kann, zerstörte im Nacktmausexperiment selektiv und effizient transplantierte humane p53^{mut} - Tumoren (Bischoff et al., 1996). Beim Menschen könnte sich allerdings eine bereits vorhandene Immunität gegenüber Adenoviren als großes Problem herausstellen. Weil der Großteil der Bevölkerung bereits durch vorangegangene Adenovirus-Infektionen immunisiert ist, ist es denkbar, daß die Mehrzahl der zur Therapie vorgesehenen Patienten das therapeutische Virus eliminieren wird, ehe es sein erwünschtes Killing-Potential entfalten kann. Aus diesem Grunde sind adenovirale Vektoren auch nur bedingt zum Einsatz in der Gentherapie geeignet.

Ausgehend von diesem Stand der Technik war es Aufgabe der vorliegenden Erfindung, Mittel und Wege bereitzustellen, um Tumorzellen gezielt bekämpfen zu können.

Erfindungsgemäß wird diese Aufgabe gelöst durch einen rekombinanten Vektor, umfassend:
(a) mindestens eine erste Transkriptionskassette, enthaltend eine für eine Rekombinase kodierende Sequenz, einen damit funktionell verbundenen Minimalpromotor MP, eine Transkriptionsfaktorbindestelle und gegebenenfalls eine Polyadenylierungssequenz, wobei der Minimalpromotor MP von der Aktivierung durch einen oder mehrere Transkriptionsfaktoren abhängig ist;
(b) mindestens eine zweite Transkriptionskassette, enthaltend ein Suizidgen, einen damit funktionell verbundenen Promotor P und gegebenenfalls eine Polyadenylierungssequenz;
(c) eine 5'-flankierende Sequenz und/oder eine 3'-flankierende Sequenz, wobei die 5'- und/oder 3'-flankierende Sequenz eine Rekombinase-Targetsequenz enthält.

Der erfindungsgemäße Vektor ermöglicht die gezielte und selektive Elimination von Tumorzellen dadurch, daß durch den Vektor ein Suizidgen in die Zellen eingeführt wird, das in gesunden Zellen durch die Expression der Rekombinase umgehend eliminiert wird, während es in Tumorzellen mit inaktiven Transkriptionsfaktoren aktiviert werden kann und durch (i) Expression des Suizidproteins, (ii) Transkription von antisense-RNA oder (iii) durch Produktion von cytopathogenem Virus zum Zelltod führt.

Im Zusammenhang mit der vorliegenden Erfindung bedeutet der Begriff "Vektor" ein lineares oder zirkuläres Nukleinsäuremolekül, das sowohl aus Desoxyribonukleinsäure als auch aus Ribonukleinsäure bestehen kann. Für die Gentherapie geeignete Vektoren, die als Ausgangsmaterial für die erfindungsgemäßen Vektoren dienen können, sind im Stand der Technik bekannt. Bevorzugte Vektoren sind dabei von viralen oder retroviralen Genomen abgeleitete Vektoren, da diese sich in Viren verpacken lassen und durch Transduktion leicht in Zellen eingebracht werden können. Vektoren auf nicht-viraler Grundlage sind ebenfalls denkbar, erfordern jedoch weitere Maßnahmen zur Transfektion. Geeignete Vektoren wären z.B. vollsynthetische Vektoren oder Vektoren, die durch attenuierte Bakterien transduziert werden.

"Replikationskompetenz" bedeutet die Fähigkeit eines Vektors, in Wirtszellen replizieren zu können. Sehr breite Replikationskompetenz in Bezug auf Säuger-zellen besitzen, z.B. Adenovirus, Retroviren wie z.B. Maus-Leukämievirus MuLV, insbesondere Moloney-Maus-Leukämie-Virus (MoMuLV). Von der Erfindung umfaßt werden allgemein in eukaryontischen Zellen replikationskompetente Vektoren. Sind die Vektoren Retroviren, so werden infektöse Retroviren bevorzugt. "Transkriptionskassetten" sind Nukleinsäureeinheiten, die neben der für ein Protein kodierenden Sequenz die erforderlichen regulatorischen Bereiche, z.B. Promotor oder Minimalpromotor mit Transkriptionsfaktorbindestelle und Polyadenylierungssequenzen, enthalten. Die erste Transkriptionskassette kann 5' oder 3' relativ zur zweiten Transkriptionskassette liegen.

Ein "Minimalpromotor" ist ein natürlicher oder synthetischer Promotor oder Enhancer, der nur in Gegenwart eines spezifischen Transkriptionsfaktors die Genexpression aktivieren kann. Er enthält mindestens eine natürliche oder synthetische Transkriptionsfaktorbindestelle.

Eine "Transkriptionsfaktorbindestelle" ist eine natürliche oder synthetische Nukleinsäuresequenz, an die ein zur Aktivierung eines Minimalpromotors erforderlicher Transkriptionsfaktor binden kann.

Eine "Rekombinase" ist ein natürliches oder synthetisches Enzym, das spezifische Targetsequenzen erkennt, schneidet und miteinander rekombiniert. Beispiele hierfür sind die Cre-Rekombinase aus dem P1-Coliphagen (Sternberg und Hamilton, 1981) und die Flp-Rekombinase aus *S*. *cerevisiae* (Broach und Hicks, 1980).

Eine "Targetsequenz" ist eine natürliche oder synthetische Sequenz, die von einer Rekombinase spezifisch erkannt werden kann. Beispiele hierfür sind die loxP-Sequenzen aus dem P1-Coliphagen (Gu et al., 1993; Sauer und Henderson, 1988; Sternberg und Hamilton, 1991) und die FRT-Sequenzen aus *S*. *cerevisiae* (Broach und Hicks, 1980; Golic und Lindquist, 1989; O'Gorman et al., 1991).

Ein "Suizidgen" ist eine Nukleinsäuresequenz, die durch Transkription und gegebenenfalls Expression zum Zelltod führt. Im erfindungsgemäßen Vektor ist es als Teil einer Transkriptionskassette, d.h. in Verbindung mit einem Promotor und gegebenenfalls einer Polyadenylierungssequenz, vorhanden.

Es ist in einem Aspekt der Erfindung beabsichtigt, daß das Suizidgen teilweise oder vollständig komplementär zu einem essentiellen Gen ist. Die durch Transkription erzeugte mRNA des Suizidgens ist fähig, mit der mRNA des essentiellen zellulären Gens zu hybridisieren. Folgen der mRNA-Hybridisierung sind Translationsarrest des essentiellen zellulären Gens und/oder RNase H-Aktivierung mit nachfolgendem Zelltod. Das essentielle zelluläre Gen kann ausgewählt sein aus Cyclinen und ati-apoptotischen Proteinen wie z.B. Bcl-2.

Ein weiterer erfindungsgemäß beabsichtigter Wirkmechanismus des Suizidgens besteht darin, daß das Suizidgen ein cytopathogenes Virus wie z.B. Semliki Forest Virus kodiert, das die Zelle abtötet. Das Suizidgen kann weiterhin eine Nukleinsäuresequenz umfassen, die ein Suizidprotein kodiert.

Ein "Suizidprotein" ist ein natürliches oder artifizielles Polypeptidprodukt, das unmittelbar oder mittelbar zum Zelltod führt. Die mittelbare Wirkung beruht z.B. auf der Wechselwirkung mit einem nicht toxischen Wirkstoffvorläufer, so daß dieser in einen toxischen Wirkstoff konvertiert wird, der den Tod einer Zelle herbeizuführen in der Lage ist. Das Suizidprotein kann ein Antigen sein wie z.B. Influenza-Hämagglutinin oder Fremd-MHC-Antigene. Die unmittelbare Wirkung beruht in diesem Fall auf der Stimulierung einer gegen die Tumorzelle gerichteten Immunantwort.

Mit den erfindungsgemäßen Vektoren ist es erstmals möglich, Tumorzellen mit defektem Transkriptionsfaktor gezielt und direkt zu eliminieren. Grundlage für die Selektivität des erfindungsgemäßen Vektors ist, daß die von der ersten Transkriptionskassette kodierte Rekombinase nur in Zellen mit intaktem Transkriptionsfaktor exprimiert werden kann. In Tumorzellen mit defektem Transkriptionsfaktor unterbleibt die Bindung des Transkriptionsfaktors an die mit dem Minimalpromotor assoziierte Transkriptionsfaktorbindestelle. Da der Minimalpromotor der Aktivierung durch den Transkriptionsfaktor bedarf, findet keine Transkription und damit auch keine Expression des von der ersten Transkriptionskassette kodierten Genes, d.h. der Rekombinase, statt. Im Gegensatz dazu kann die kodierende Sequenz der zweiten Transkriptionskassette, d.h. das Suizidgen, transkribiert werden, da der mit diesem Gen funktionell verbundene Promotor von einer Aktivierung durch einen Transkriptionsfaktor unabhängig ist. Das nunmehr exprimierte Suizidprotein wird, nachdem es in Kontakt mit einem Wirkstoffvorläufer gekommen ist, diesen metabolisieren und damit zur Produktion eines toxischen Wirkstoffs beitragen. Dieses Metabolismusprodukt führt nachfolgend zum Tod der jeweils betroffenen Zelle. Alternativ könnte unmittelbar eine zelltypspezifische antisense-RNA oder ein Virus zum Zelltod führen.

In gesunden Zellen dagegen, d.h. in Zellen mit intaktem Transkriptionsfaktor, kann dieser an die Transkriptionsfaktorbindestelle binden, so daß das von der ersten Transkriptionskassette kodierte Protein, d.h. die Rekombinase, exprimiert wird. Aufgrund der z.B. a priori vom Vektor mitgebrachten Rekombinase-Targetsequenzen, die den Bereich der Transkriptionskassetten flankieren, wird der zwischen den Targetsequenzen liegende Bereich mit der ersten und der zweiten Transkriptionskassette sofort deletiert. Im Fall von retroviralen Vektoren wird durch die Integration eine Verdoppelung des LTR's und somit eine Verdoppelung der Rekombinase-Targetsequenz(en) herbeigeführt. Der zwischen den Targetsequenzen liegende Bereich wird ebenfalls deletiert. Zurück bleibt im Fall einer gesunden Zelle lediglich eine Kopie der Targetsequenz gegebenenfalls zusammen mit dem LTR pro vormaliger Integrationsstelle des Vektors.

Bei dem angesprochenen Transkriptionsfaktor kann es sich um jeden Transkriptionsfaktor mit einem Defekt handeln, der eine Einschränkung oder vollständige Beseitigung der Transaktivatorfähigkeit zur Folge hat. Ein bekanntes Beispiel für einen Transkriptionsfaktor, dessen DNA-bindende Domäne in Tumorzellen relativ häufig so verändert ist, daß eine DNA-Bindung und folglich auch eine Transaktivierung nicht mehr stattfindet, ist der erwähnte Faktor p53.

In humanen Tumoren befinden sich die p53-Mutationen immer in der DNA-Bindungsdomäne. Dies führt häufig zu einem Verlust des DNA Bindungsvermögens und damit der Transaktivatorfunktion des Proteins. p53 bindende Konsensussequenzen wurden in einer Reihe von Promotoren gefunden und charakterisiert. p53 reguliert darüber unter anderem die Expression von p21 (WAF1) (el-Deiry et al. 1994), bax (Miyashita and Reed, 1995) und IGF-BP3 (Buckbinder et al. 1995). Eine derartige p53-bindende Konsensussequenz ist z.B. das PG-Motiv (CCTGCCTGGACTTGCCTG) (el-Deiry et al, 1992). Die Erfinder und andere haben gezeigt, daß dieses Motiv (PGₙ) in Zusammenhang mit einem Minimalpromotor (MP) die Expression eines Reportergens p53-induzierbar macht. Eine derartige Induktion ist mit mutiertem p53 nicht möglich. Dies wurde unter anderem für die Kombinationen PGₙ-CMVMin-CAT (Chloramphenicol-Acetyl-Transferase) (Kern et al., 1992) und pg13-SV40Min-SEAP (Sekretierte-Alkalische-Phosphatase) gezeigt. Die p53-bindende Konsensussequenz pg13 ist für die Zwecke der Erfindung bevorzugt.

In einer Ausführungsform der Erfindung bestehen die flankierenden Sequenzen im wesentlichen aus den Rekombinase-Targetsequenzen. Bei diesen kann es sich um natürliche oder synthetische Sequenzen handeln, die von einer natürlichen oder rekombinanten Rekombinase erkannt werden.

In einer bevorzugten Ausführungform ist der erfindungsgemäße Vektor von einem Retrovirus abgeleitet. Retroviren sind RNA-Viren, deren Replikation ein DNA-Intermediat involviert. Das virale RNA-Genom wird von kurzen wiederholten Sequenzen (Repeats, R) und nicht wiederholten Sequenzen (Unique Sequences, U5 und U3) flankiert, die die DNA-Synthese, die Integration des Virusgenoms in das Wirtsgenom, die Transkription und die RNA-Prozessierung steuern. Zwischen diesen Kontrollbereichen befinden sich kodierende Sequenzen für die wichtigsten Strukturproteine des Viruspartikels, nämlich gag und env, und für weitere im Partikel verpackte Enzyme (pol, Protease, reverse Transkriptase und Integrase). Kurz nach der Infektion wird die virale RNA von der reversen Transkriptase in DNA übersetzt. Vor der Integration werden die endständigen Sequenzen des viralen Genomes verdoppelt, so daß das retrovirale Genom von langen endständigen wiederholten Sequenzen (Long Terminal Repeats, LTR) flankiert wird, die jeweils einen U3-Bereich, R und einen U5-Bereich enthalten. Die linearen Moleküle werden dann in das Genom integriert.

Nach Integration des revers transkribierten Virusgenoms in das Wirtsgenom spricht man von einem Provirus. Das Provirus wird zusammen mit der zellulären Wirts-DNA repliziert und wie ein zelluläres Gen transkribiert. Die Provirustranskription wird durch Promotor- und Enhancersequenzen gesteuert, die sich im U3-Bereich des 5'-LTR befinden. Polyadenylierte Transkripte beginnen an dem Übergang zwischen U3 und R im 5'-LTR und enden im R des 3'-LTR, welcher das Polyadenylierungssignal enthält.

Vorausgesetzt, daß bestimmte Kontrollsequenzen innerhalb der LTRs erhalten bleiben, kann das retrovirale Genom gegen Fremd-DNA ausgetauscht werden, ohne seine Fähigkeit zur Replikation in Zellen, die die für die reverse Transkription, Integration und Partikelbildung notwendigen Proteine exprimieren, zu beeinträchtigen. Um die für die Replikationsmaschinerie erforderlichen Enzyme bereitzustellen, wird Vektor-DNA in Zellinien transfiziert, die entweder vollständige retrovirale Genome oder Helferviren enthalten. Die Helferviren können sich dabei in Folge einer Deletion zwischen U5 und gag nicht zu Partikeln zusammenlagern. Infolgedessen werden nur rekombinante Transkripte verpackt und von den Zellen in Viruspartikeln freigesetzt. Aufgrund dieser technischen Möglichkeiten sind retrovirale Vektoren als Vektoren für die Gentherapie bevorzugt.

Im Fall eines von Retroviren abgeleiteten Vektors umfassen die 5'- und/oder 3'-flankierenden Sequenzen vollständige oder partielle LTR-Bereiche. Die mindestens eine Rekombinase-Targetsequenz befindet sich dabei bevorzugt im U3- oder U5-Bereich des 5'- und/oder 3'-LTRs. Liegt die Rekombinase-Targetsequenz außerhalb des LTR, so sind mindestens 2 Rekombinase-Targetsequenzen erforderlich. Die Transkriptionskassetten jedoch befinden sich bevorzugt außerhalb der LTR's. Im Fall nicht retroviraler Vektoren werden mindestens zwei Rekombinase-Targetsequenzen zur Verfügung gestellt.

Die erste Transkriptionskassette enthält u.a. eine für eine Rekombinase kodierende Sequenz. Sie wird bevorzugt in gleicher Transkriptionsrichtung wie das gegebenenfalls zugrundeliegende Virusgenorm eingeordnet.

Als Rekombinase kann jedes natürliche oder synthetische Protein mit Rekombinaseaktivität verwendet werden. In einer bevorzugten Ausführungsform ist die von der ersten Transkriptionskassette kodierte Rekombinase die Rekombinase Cre. Die Cre-Rekombinase wurde ursprünglich im Coliphagen P1 identifiziert und ist sehr gut charakterisiert. In bevorzugten Ausführungsformen ist die Rekombinase-Targetsequenz für die Rekombinase Cre die loxP-Sequenz. Artifizielle Rekombinase-Targetsequenzen, die von der Rekombinase Cre erkannt werden, können ebenfalls verwendet werden. In einer alternativen Ausführungsform wird als Rekombinase Flp eingesetzt. Flp stammt ursprünglich aus *S*. *cerevisiae*. Sie erkennt bevorzugt FRT-Sequenzen aus *S*. *cerevisiae*. Diese Targetsequenzen könnten wiederum durch artifizielle Targetsequenzen, die von der Rekombinase Flp erkannt werden, ersetzt werden.

Als Minimalpromotor kann jeder von einem Transkriptionsfaktor abhängige Promotor verwendet werden. Bevorzugte Ausführungsformen sehen die Verwendung von ΔCMV (Gossen & Bujard, 1992) oder ΔMMTV (Hoffmann et al., 1997) vor. Bei diesen Promotoren handelt es sich um trunkierte Cytomegalie- bzw. Maus-Mammatumorvirus-Promotoren, die von einer Transaktivierung durch Transkriptionsfaktoren abhängig sind.

Das von der zweiten Transkriptionskassette kodierte Suizidgen ist in einer bevorzugten Ausführungsform ein Thymidinkinasegen. Erfindungsgemäß besonders bevorzugt ist dabei Thymidinkinase aus Herpes-Simplex-Virus (HSV-TK). HSV-TK setzt z.B. zugefügtes Ganciclovir zu einem toxischen Metabolismusprodukt um. Als Suizidproteine können jedoch alle Proteine vorgesehen werden, von denen bekannt ist, daß sie nach Metabolisierung aus einem nicht toxischen Vorläuferwirkstoff einen toxischen Wirkstoff erzeugen. Ein weiteres Beispiel eines solchen Suizidproteines ist die Cytosindesaminase.

Das Suizidgen ist in einer weiteren bevorzugten Ausführungsform teilweise oder vollständig komplementär zu einem essentiellen zellulären Gen. Die durch Transkription des Suizidgens erzeugte mRNA ist fähig, mit der mRNA des essentiellen zellulären Gens zu hybridisieren (antisense-Mechanismus). Folgen der mRNA-Hybridisierung sind Translationsarrest und/oder RNase H-Aktivierung, welches die Expression des essentiellen zellulären Gens verhindert und zum Zelltod führt. Essentielle zelluläre Gene wie z.B. des Primärstoffwechsels sind dem Fachmann bekannt.

Das Suizidgen ist in einer weiteren bevorzugten Ausführungsform in der Lage, durch Expression ein cytopathogenes Virus herzustellen. Dem Fachmann bekannte cytopathogene Viren erzeugen in den infizierten Zellen degenerative Veränderungen wie z.B. Bildung von Riesenzellen, Syncytien, Einschlußkörpern, Vakuolen und Granula, Veränderungen des Zellkerns und Lyse der Zellen. Ein beispielhaftes cytopathogenes Virus ist das Semliki Forest Virus.

Die zweite Transkriptionskassette wird bevorzugt in gleicher Transkriptionsrichtung wie das Virusgenom und die erste Transkriptionskassette angeordnet, wobei die erste Transkriptionskassette 5'oder 3' relativ zur zweiten Transkriptionskassette angeordnet sein kann. Bei entgegengesetzter Anordnung, die auch möglich ist, muß für die zweite Transkriptionskassette eine weitere Polyadenylierungssequenz vorgesehen werden.

Als Promotor für das Suizidgen kann jeder beliebige starke eukaryontische Promotor eingesetzt werden. Bevorzugte Promotoren sind beispielsweise der LTR-Promotor des Moloney-Maus-Leukämievirus (MoMuLV). Dem Fachmann sind weitere Promotoren bekannt.

In einer weiteren bevorzugten Ausführungsform enthalten die erfindungsgemäßen Vektoren außerdem eine Selektionsmarkerkassette. Selektionsmarkerkassetten können z.B. in eine U3-Region inseriert werden. Diese kann als Nachweis für die erfolgte Integration verwendet werden. Eine in den Beispielen dargestellte Selektionsmarkerkassette ist SV40Puro. Jedes andere in Eukaryonten transkribierte und translatierte, dem Fachmann bekannte Selektionsmarkersystem kann jedoch ebenso verwendet werden.

Die Erfindung bezieht sich weiterhin auf pharmazeutische Zusammensetzungen, die den Vektor nach einem der Ansprüche 1 bis 14 oder 16 enthalten. Diese pharmazeutischen Zusammnensetzungen sind nützlich für die Therapie von Tumoren, die sich durch die Defizienz eines Transkriptionsfaktors auszeichnen. In bevorzugten Ausführungsformen liegt der Vektor in der therapeutischen Zusammensetzung in einem Viruspartikel verpackt vor.

Bevorzugt sind die erfindungsgemäßen Vektoren in den pharmazeutischen Zusammensetzungen replikationskompetent. Besonders bevorzugt sind sie infektös, so daß sie in der Lage sind, weitere Zellen zu infizieren.

Der erfindungsgemäße Vektor kann verwendet werden, um Tumorpatienten zu behandeln. In einer bevorzugten Ausführungsform ist es beabsichtigt, durch Verabreichen des erfindungsgemäßen Vektors und anschließende Gabe einer durch das Suizidprotein metabolisierbaren Vorläufersubstanz, z.B. Ganciclovir, gezielt Tumorzellen abzutöten.

Weiterer Gegenstand der Erfindung ist eine transduzierte eukaryontische Zelle, die durch Infizieren einer Tumorzelle, die einen defizienten Transkriptionsfaktor aufweist, mit einem rekombinanten Vektor nach einem der Ansprüche 1 bis 14 oder 16 erhalten werden kann.

Die folgenden Figuren und die Beispiele erläutern die Erfindung.

### Kurze Beschreibung der Figuren

### Figur 1:

Vektorkonzept und Funktionsprinzip zur selektiven Eliminierung von Tumorzellen. P= Promotor, MP = Minimalpromotor, pA = Polyadenylierungssequenz, lx = loxP Targetsequenz für die sequenzspezifische Rekombinase Cre.

### Figur 2:

Selbstdeletierende retrovirale Vektoren und Funktionsprinzip zur selektiven Eliminierung von Tumorzellen. Nach Integration des Provirus befinden sich Suizidgen und Cre-Rekombinase zwischen zwei loxP Stellen. In normalen Zellen wird die Expression der Cre-Rekombinase aktiviert und der Großteil der integrierten Sequenzen eliminiert. In Krebszellen hingegen liegt der Transkriptionsfaktor mutiert vor und kann die Transkription der Rekombinase nicht aktivieren. Als Folge davon exprimieren nur infizierte Krebszellen das Suizidgen. Auf diese Weise werden nur Tumorzellen gegenüber dem entsprechenden Wirkstoff empfindlich. U3RU5 = retrovirale Kontrollregion mit Promotor, MP = Minimalpromotor, lx = loxP Targetsequenz für die sequenzspezifische Rekombinase Cre.

### Figur 3:

Selbstdeletierende retrovirale Vektoren zur selektiven Eliminierung von Tumorzellen durch Ganciclovir. HSV-TK = Herpes simplex virus 2 Thymidin-kinasegen, pg13 = p53-bindende Konsensussequenz, CMV = Cytomegalievirus-Promotor, MMTV = Maus-Mammatumorvirus-Promotor, lx = loxP Targetsequenz für die sequenzspezifische Rekombinase Cre, SV40 = Simian virus 40-Promotor, Puro = Puromycinresistenzgen.

### Figur 4:

Unterschiedliches Verhalten der selbstdeletierenden Retroviren in normalen und Tumorzellen. Nach Verdopplung der U3-Region des LTR nach Integration der Proviren liegt der Großteil der Konstrukte zwischen zwei loxP Stellen. p53 abhängige Transkription des Cre-Enzyms führt zur selektiven Rekombination in normalen Zellen. In Tumorzellen liegt p53 mutiert und damit nicht funktionell vor. Eine Cre-vermittelte Rekombination ist dadurch nicht möglich und das gesamte Provirus bleibt erhalten. Als Folge davon wird HSV-TK exprimiert und macht die Tumorzellen empfindlich auf Ganciclovir. Normale Zellen reagieren aufgrund der fehlenden HSV-TK nicht auf Ganciclovir. HSV-TK = Herpes simplex virus 2 Thymidin-kinasegen, pg13 = p53-bindende Konsensussequenz, CMV = Cytomegalievirus Promotor, MMTV = Maus-Mammatumorvirus-Promotor, lx = loxP Targetsequenz für die sequenzspezifische Rekombinase Cre, SV40 = Simian virus 40 Promotor, Puro = Puromycinresistenzgen.

### Figur 5:

Cre-Reporterplasmid pSVpax1. Cre-vermittelte Rekombination führt zur Transkription des LacZ-Gens vom SV40 Promotor. SV = SV40 Promotor, x = loxP Targetsequenz für die sequenzspezifische Rekombinase Cre, Puro = Puromycinresistenzgen, LacZ = β-Galaktosidasegen, pA = Polyadenylierungssequenz.

### Figur 6:

Transfektion der p53-abhängigen Cre-Expressionsplasmide in die Reporter-Zellinie Saos2/pSVpax1: A: Transfektion von ppg13ΔCMVCrepA allein (-p53) bzw. mit pSBC2-p53 (+p53). B: Kotransfektion von ppg13ΔMMTVCrepA allein (-p53) bzw mit pSBC2-p53 (+p53).

### Figur 7:

PCR zum Nachweis der Cre-Rekombinase. Genomische DNA wurde mit Cre-spezifischen Primern amplifiziert. Als Kontrolle der Provirusintergration dienten SV40-spezifische Primer.

### BEISPIELE

### Beispiel 1

### Herstellung einer Saos2-Reporterzellinie zum Nachweis von Cre-vermittelter Rekombination

Um eine Zellinie zu erhalten, mit der die Aktivität der Cre-Rekombinase nachgewiesen werden kann, wurden humane, p53-defiziente Saos2-Osteosarkomzellen mit dem Reporterkonstrukt pSVpaX1 mittels Ca-Phosphat-Kopräzipitations-Methode (Pear et al., 1993) stabil transfiziert.

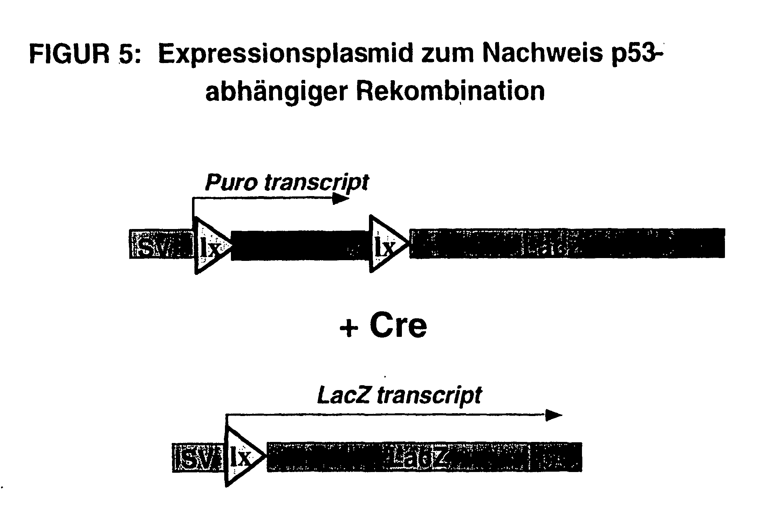

Das Reporterkonstrukt pSVpaX1 (Buchholz et al., 1996) besteht aus dem LacZ-Gen, das für ein leicht detektierbares Protein (β-Galaktosidase) kodiert. Das LacZ-Gen wird erst nach Cre-vermittelter Rekombination von einem SV40-Promotor transkribiert (Figur 5).

Zwischen den kodierenden Sequenzen des SV40-Promotors und des LacZ-Gens befindet sich ein Puromycin-Phosphotransferase-Resistenzgen, das von zwei loxP Stellen flankiert wird. Der SV40-Promotor transkribiert im nicht-rekombinierten Zustand das Resistenzgen und ermöglicht die Selektion auf das Reporterkonstrukt. In Anwesenheit von Cre-Rekombinase werden die Puromycin-Phophotransferase-Sequenzen zwischen den loxP-Stellen entfernt und das LacZ-Gen unter die Kontrolle des SV40-Promotors gebracht. Durch Detektiön der β-Galaktosidase-Expression in den Zellen (X-Gal Färbung) ist somit ein einfacher Nachweis der Cre-Rekombinase Aktivität möglich.

Die mit pSVpaX1 transfizierten Zellen wurden für 7 Tage mit 0.5 mg/ml Puromycin (Sigma) selektiert. Die erhaltenen Transformanten zeigten keinerlei Hintergrund-Färbung nach Inkubation mit X-Gal. Die Transfektion mit dem Cre-Expressionsplasmid pMC-Cre führte zu einem deutlichen LacZ-positiven Phänotyp der transfizierten Zellen.

### Beispiel 2

### Herstellung von p53-abhängigen Cre-Expressionsplasmiden

Die p53-Abhängigkeit zeigten wir für zwei verschiedene Minimalpromotoren mit Hilfe von Cre-Expressionsplasmiden. Diese Minimalpromotoren bestehen aus dem p53-bindenden pg13-Motiv (Kern et al., 1992), gekoppelt zum einen an einen verkürzten Cytomegalie-Virus (ΔCMV)-Promotor (Gossen and Bujard, 1992), zum anderen an einen verkürzten murinen Mammatumor-Virus (ΔMMTV)-Promotor (Hoffmann et al., 1997). Die Klonierung der beiden Expressionsplasmide geschah auf folgendem Weg:
1. Verdau von pBluescript (pBS) mit dem Restriktionsenzym Clal und dem Klenow-Enzym und von pU3Cre (Russ et al., 1996) mit Nhel und Klenow-Enzym. Ligation des Cre-Fragmentes mit dem linearisierten Vektor ergab pBSCre.
2. Verdau von pBSCre und von pGLpg13Seap (C. Rinderele, Asta Medica AG) mit Sacl und EcoRI. Das pg13-Fragment wurde als Sacl/EcoRI-Fragment aus pGlpg13Seap 5' von Cre in pBSCre kloniert. Dies ergab ppg13Cre.
3. Verdau von ppg13Cre mit HindIII und Klenow-Enzym. Ligationen: 1. mit dem synthetischen ΔCMV-Promotor Stück (GGC CGG CCT ATA AGC AGA GCT CGT TTA GTG GCC) ergab ppg13ΔCMVCre. 2. mit einem PCR-Fragment des ΔMMTV-Promotors (Primer 5': CCT ATG TTA TTT TGG AAC TTA TCC, Primer 3': AGG GCC CTG TTC GGG CGC C) ergab ppg13ΔMMTVCre.
4. Verdau der beiden Konstrukte (ppg13ΔCMVCre und ppg13ΔCMVCre) mit Sall und Klenow-Enzym und von ppg13Seap mit BamHI, Sall und Klenow-Enzym. Ligation des ausgeschnittenen Poly-Adenylierungs-Signals (pA) resultierte in den zwei folgenden p53-abhängigen Cre-Expressionsplasmiden:
   _ ppg13ΔCMVCrepA
   _ ppg13ΔMMTVCrepA.

Zum Nachweis der p53-abhängigen Cre-vermittelten Rekombination wurden die Cre-Expressionsplasmide transient mit und ohne das p53-Expressionsplasmid pSBC2-p53 in die p53^{-/-}- Reporter-Zellinie Saos2/pSVpax1 transfiziert. Die Transfektion geschah nach einem Standardprotokoll mittels Ca-Phosphat Kopräzipitation (Pear et al., 1993).

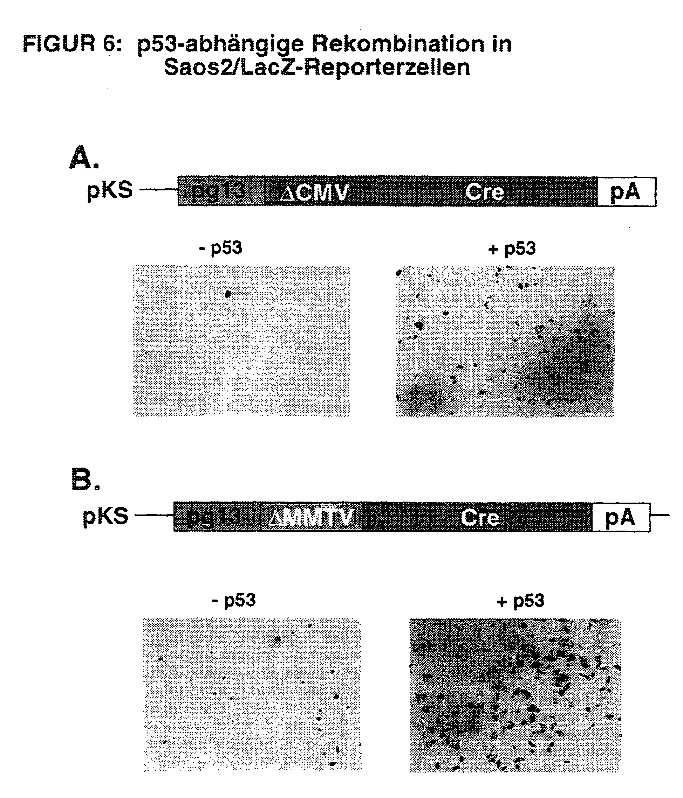

Nach 48 Stunden wurden die Zellen zum Nachweis von β-Galaktosidase mit X-Gal gefärbt. Figur 6 zeigt in beiden Fällen deutlich, daß p53 die Cre-vermittelte Rekombination des Reporterplasmids pSVpax1 und als Folge davon die Expression von β-Galaktosidase signifikant stimulierte.

Die Ergebnisse belegen, daß die beiden erzeugten Minimalpromotoren pg13ΔCMV und pg13ΔMMTV eine p53-abhängige Expression der Cre Rekombinase ermöglichen.

### Beispiel 3

### Klonierung von p53-regulierten selbstdeletierenden retroviralen Vektoren

Der Klonierungsweg zu den p53-abhängigen selbstdeletierenden Konstrukten ist im folgenden kurz dargestellt:
1. Verdau von pBS und von pGLpg13Seap mit HindIII. Ligation des Vektors mit dem pg13SV40 Fragment ergab ppg13SV40.
2. Verdau von pU3TK (Russ et al. 1996b) mit Nhel und Klenow-Enzym und von ppg13SV40 mit Sall und Klenow-Enzym. Ligation der Thymidinkinase (TK)-Kassette mit dem Vektor ergab pHSVTKpgSV40.
3. Verdau von pU3Cre mit Nhel und Klenow-Enzym und von pTKpg13SV40 mit Xbal und Klenow-Enzym. Ligation von Cre mit dem Vektor ergab pHSVTKpgSV40Cre.
4. Verdau von pTKpg13SV40Cre mit Notl, Klenow-Enzym, danach mit Xhol, und von pBABEpuro (Russ et al. 1996b) mit Clal, Klenow-Enzym und danach mit Sall. Ligation der TKpg13SV40Cre-Kassette mit dem pBABE-Fragment ergab pHSVTKpgSV40CreU3RU5.
5. Die lxSV40puro-Kassette aus dem Konstrukt pggSV40CrelxSV40Puro (Russ et al., 1996a) wurde mit BamHI und Clal ausgeschnitten und in die entsprechenden Restriktionsstellen von pBS ligiert. Das ergab pBSlxSV40Puro.
6. Verdau von pHSVTKpgSV40CreU3RU5 mit Nhel und pBSlxSV40Puro mit Spel und Xbal. Ligation der lxSV40Puro-Kassette mit dem retroviralen Vektor ergab pHSVTKpgSV40CreU3lxSV40Puro.

Der Minimalpmmotor pg13SV40 wurde wie folgt gegen die Minimalpromotoren pg13ΔCMV bzw. pg13ΔMMTV ausgetauscht:

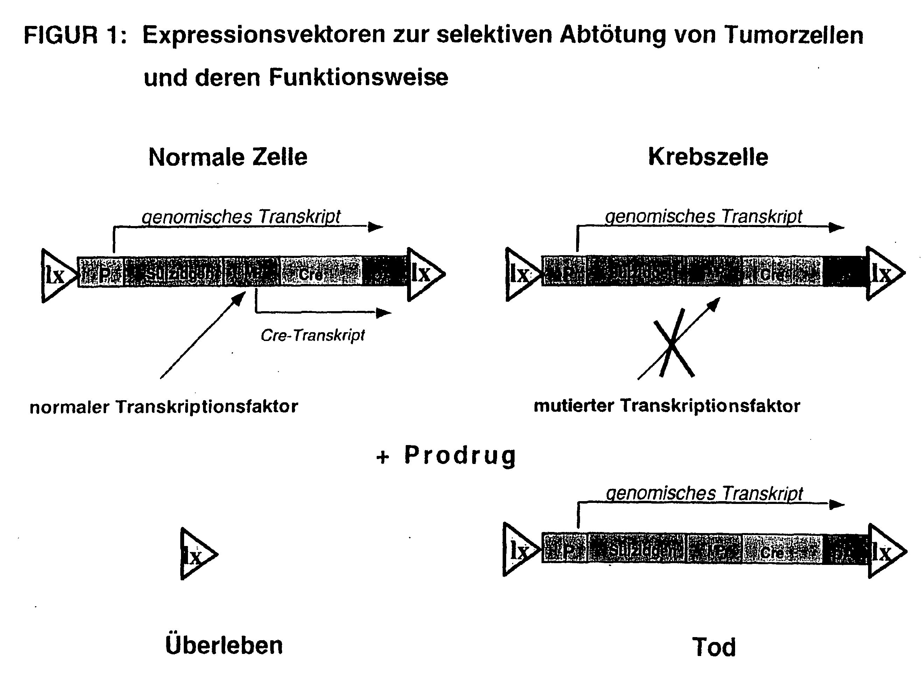

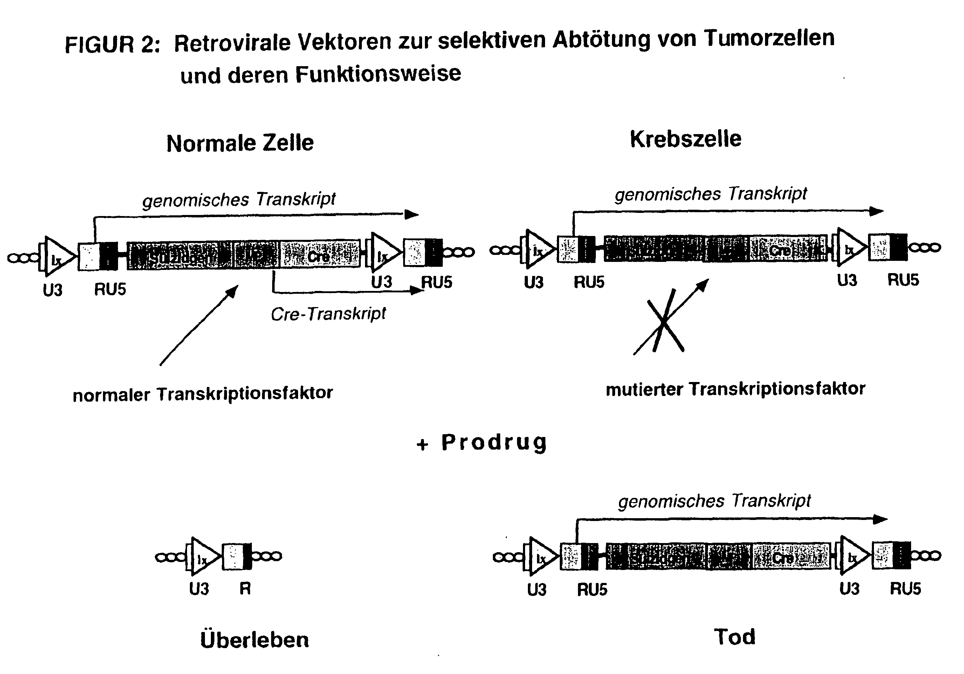

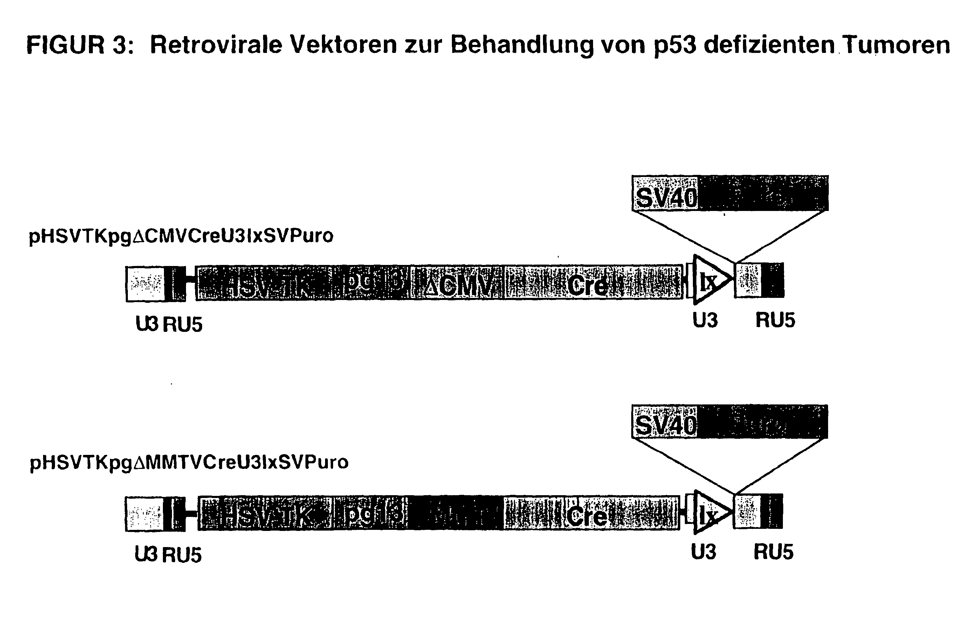

Verdau des Plasmides pHSVTKpgSV40CreU31×SV40Puro mit BstBI und partieller Verdau mit EcoRI. Verdau der Expressionsplasmide ppg13ΔCMVCrepA bzw. ppg13ΔMMTVCrepA mit denselben Restriktionsenzymen. Ligation der entsprechenden Fragmente ergab die beiden folgenden p53-regulierten selbstdeletierenden Konstrukte (Figur 3):
_ pHSVTKpgΔCMVCreU3IxSV40Puro
_ pHSVTKpgΔMMTVCreU3IxSV40Puro.

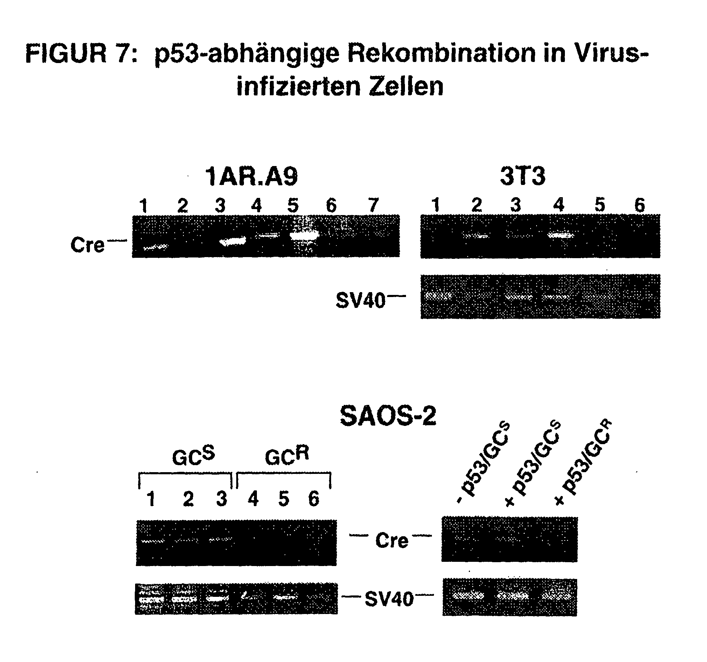

Zum Nachweis, daß die Cre/loxP vermittelte Rekombination auch in den retroviralen Vektoren pHSVTKpgΔCMVCreU3lxSV40Puro und pHSVTKpgΔMMTVCre-U3lxSV40Puro p53-abhängig bleibt, wurden die Plasmide transient in Phoenix Zellen transfiziert und in retrovirale Partikel verpackt (Pear et al. in press und Internet Web page). Mit den dadurch erzeugten virushaltigen Zellkultur-Überständen infizierten wir Saos2-Zellen, welche kein p53 enthalten. Nach Selektion in Puromycin (5 µg/ml) konnten wir Provirus-enthaltende Einzelklone isolieren. Zum Nachweis einer möglicherweise undichten Cre-Expression in Abwesenheit von p53 wurden einzelne Klone in 20 µM Ganciclovir (GC, Syntex) gehalten. Tabelle 1 zeigt, daß in Abhängigkeit vom jeweiligen Minimalpromotor 56% bzw. 83% der Provirus-exprimierenden Klone in GC starben. In sämtlichen GC sensitiven Klonen konnte Cre DNA mittels spezifischer PCR nachgewiesen werden (Figur 7). Dies bedeutet, daß in der Mehrzahl der infizierten Saos2 Klone keine Rekombination stattgefunden hat. In weiteren Versuchen wurden GC sensitive Klone mit dem Wildtyp-p53 Expressionsplasmid (pSBC2-p53) transient transfiziert. GC überlebende Zellen wurden wieder mittels PCR auf Rekombination untersucht. Wie in Figur 7 beispielhaft dargestellt, enthielten die GC resistenten Zellen keine Cre-Rekombinase mehr. Diese Ergebnisse belegen eindeutig, daß Wildtyp-p53 in der Lage ist, die zwischen den proviralen LTRs positionierten Sequenzen aus dem Genom zu eliminieren, indem es die Transkription der Cre-Rekombinase aktiviert.

### Beispiel 4

### Selektive Abtötung von p53^{-/-}-Zellen durch selbstdeletierende retrovirale Vektoren

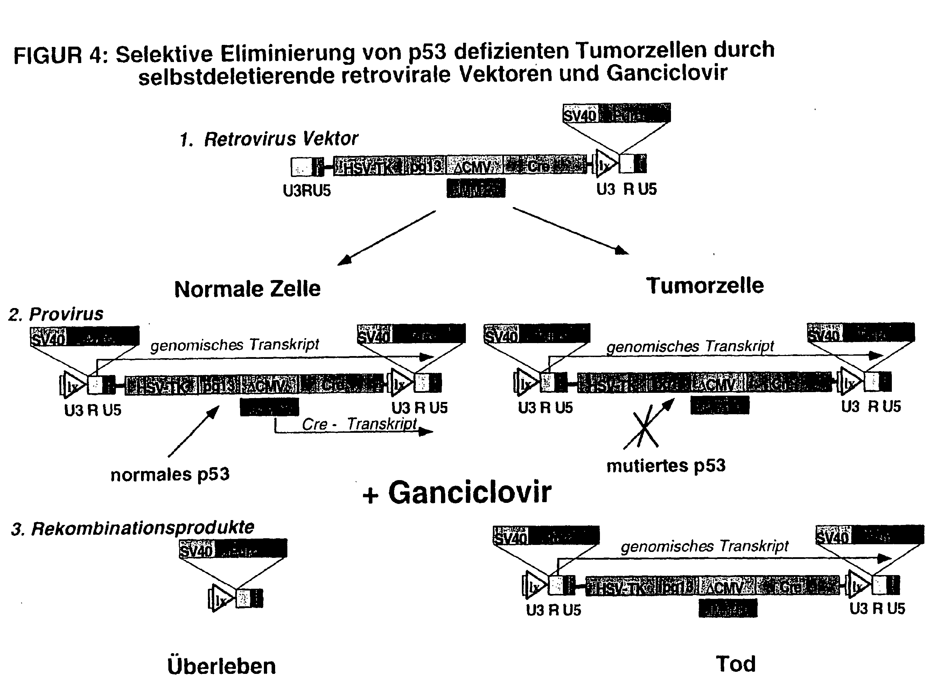

Zur Beantwortung der Frage, ob endogenes p53 ebenfalls in der Lage ist, die Cre Expression der Vektoren zu aktivieren, wurden, wie in Beispiel 3 beschrieben, transformierte Mausfibroblasten mit den gleichen Retroviren infiziert und Provirus enthaltende Klone isoliert. Bei den Fibroblasten handelte es sich zum einen um p53^{-/-}-1AR.A9 Zellen, welche aus p53 knock-out Mäusen stammen (Lowe et al., 1994), zum anderen um konventionelle NIH3T3 Zellen, die geringe Mengen Wildtyp-p53 exprimieren. Während 100% aller Puromycin-resistenten 1AR.A9-Klone aufgrund fehlender Rekombination in GC starben, überlebten 57% der NIH3T3 Zellklone (Figur 7, Tabelle 1). DNA - Untersuchungen zeigten, daß diesem Phänotyp eine Cre-vermittelte Rekombination zugrunde lag, welche den Großteil des Provirus einschließlich der HSVTK eliminierte (Figur 4).

**Tabelle 1: p53-abhängige Ganciclovir-Resistenz in Virus-infizierten Zellen***

| | | | | | | |
|---|---|---|---|---|---|---|
| Zellen | 1AR.A9 | | NIH3T3 | | Saos 2 | |
| Minimal-promoter | ΔCMV | ΔMMTV | ΔCMV | ΔMMTV | ΔCMV | ΔMMTV |
| Überlebende Klone | 0/5 (0%) | 0/10 (0%) | nt | 4/7 (57%) | 9/16 (56%) | 15/18 (83%) |

| | | | | | | |
|---|---|---|---|---|---|---|
| *Puromycin-resistente Klone wurden in Gegenwart von Ganciclovir (20 mM) gezüchtet und die Anzahl der überlebenden Klone nach 7-tägiger Inkubation bestimmt. | | | | | | |
| nt: nicht untersucht | | | | | | |

### LITERATURLISTE

Bischoff, J. R., D. H. Kirn, A. Williams, C. Heise, S. Horn, M. Muna, L. Ng, J. A. Nye, A. Sampson-Johannes, A. Fattaey, and F. McCormick. 1996. An adenovirus mutant that replicates selectively in p53-deficient human tumor cells [see comments]. *Science* 274, 373-6.
Broach, J. R., and J. B. Hicks. 1980. Replication and recombination functions associated with the yeast plasmid, 2µ circle. *Cell* 21, 501-508.
Buchholz, F., L. Ringrose, P. O. Angrand, F. Rossi, and A. F. Stewart. 1996. Different thermostabilities of FLP and Cre recombinases: implications for applied site-specific recombination. *Nucleic Acids Res* 24, 4256-62.
Buckbinder, L., R. Talbott, S. Velasco-Miguel, I. Takenaka, B. Faha, B. R. Seizinger, and N. Kley. 1995. Induction of the growth inhibitor IGF-binding protein 3 by p53. *Nature* 377, 646-9
Donehower, L. A., M. Harvey, B. L. Slagle, M. J. McArthur, C. A. Montgomery, Jr., J. S. Butel, and A. Bradley. 1992. Mice deficient for p53 are developmentally normal but susceptible to spontaneous tumours. *Nature* 356, 215-21.
el-Deiry, W. S., S. E. Kern, J. A. Pietenpol, K. W. Kinzier, and B. Vogelstein. 1992. Definition of a consensus binding site for p53. *Nat Genet* 1, 45-9.
el-Deiry, W.S., J. W. Harper, P. M. O'Connor, V. E. Velculescu, C. E. Canman, J. Jackman, J. A. Pietenpol, M. Burell, D. E. Hill, Y. Wang, and et al. 1994. WAF1/CIP1 is induced in p53-mediated G1 arrest and apoptosis. *Cancer Res* 54, 1169-74.
Golic, K. G., and S. Lindquist. 1989. The FLP recombinase of yeast catalyzes site-specific recombination in the Drosophila genome. *Cell* 59, 499-509.
Gossen, M., and H. Bujard. 1992. Tight control of gene expression in mammalian cells by tetracycline-responsive promoters. *Proc Natl Acad Sci U S A* 89, 5547-5551.
Gu, H., Y. Zou, and K. Rajewsky. 1993. Independent control of immunoglobulin switch recombination at individual switch regions evidenced through Cre-*loxP* - mediated gene targeting. *Cell* 73, 1155-1164.
Hoffmann, A., M. Villalba, L. Journot, and D. Spengler. 1997. A novel tetracycline-dependent expression vector with low basal expression and potent regulatory properties in various mammalian cell lines. *Nucleic Acids Res* 25, 1078-1079.
Kern, S., J. Pietenpol, S. Thiagalingam, A. Seymour, K. Kinzler, and B. Vogelstein. 1992. Oncogenic forms of p53 inhibit p53-regulated gene expression. *Science* 256, 827-830.
Levine, A. J. 1997. p53, the cellular gatekeeper for growth and division. *Cell* 88, 323-31.
Levine, A. J., J. Momand, and C. A. Finlay. 1991. The p53 tumour suppressor gene. *Nature* 351, 453-6.
Lowe, S., S. Bodis, A. McClatchey, L. Remington, H. Ruley, D. Fisher, D. Housman, and T. Jacks. 1994. p53 status and the efficacy of cancer therapy in vivo. *Science* 266, 807-810.
Lowe, S. W., H. E. Ruley, T. Jacks, and D. E. Housman. 1993. p53-dependent apoptosis modulates the cytotoxicity of anticancer agents. *Cell* 74, 957-67.
Miyashita, T., and J. C. Reed. 1995. Tumor suppressor p53 is a direct transcriptional activator of the human bax gene. *Cell* 80, 293-9
O'Gorman, S., D. T. Fox, and G. M. Wahl. 1991. Recombinase-mediated gene activation and site-specific integration in mammalian cells. *Science* 251, 13511355.
Pear, W., G. Nolan, M. Scott, and D. Baltimore. 1993. Production of high-titer helper-free retroviruses by transient transfection. *Proc Natl Acad Sci USA* 90, 8392-8396.
Pear, W., M. Scott, and G. Nolan. Rapid production of high titer, helper-free retroviruses using transient transfection. *Methods in Gene Therapy* in press und Internet Web page: http://www.stanford.edu/group/nolan/NL-phoenix.html
Russ, A., C. Friedel, K. Ballas, K. U, D. Zahn, K. Strebhardt, and H. v. Melchner. 1996a. Identification of genes induced by factor deprivation in hematopoietic cells undergoing apoptosis using gene-trap mutagenesis and site-specific recombination. *Proc Natl Acad Sci U S A* 93, 15279-15284.
Russ, A., C. Friedel, M. Grez, and H. v. Melchner. 1996b. Self-deleting retrovirus vectors for gene therapy. *J Virol* 70, 4927-4932.
Sauer, B., and N. Henderson. 1988. Site-specific recombination in mammalian cells by the Cre recombinase of bacteriophage P1. *Proc. Natl. Acad. Sci. USA* 85, 5166-5170.
Sternberg, N., and D. Hamilton. 1981. Bacteriophage P1 site-specific recombination. I. Recombination between loxP sites. *J*. *Mol. Biol*. 150, 467-486.

## Patentansprüche

1. Rekombinanter Vektor, umfassend:
(a) mindestens eine erste Transkriptionskassette, enthaltend eine für eine Rekombinase kodierende Sequenz, einen damit funktionell verbundenen Minimalpromotor MP, eine Transkriptionsfaktorbindestelle und gegebenenfalls eine Polyadenylierungssequenz, wobei der Minimalpromotor MP von der Aktivierung durch einen oder mehrere Transkriptionsfaktoren abhängig ist;
(b) mindestens eine zweite Transkriptionskassette, enthaltend eine Nukleinsäuresequenz, die durch Transkription und gegebenenfalls Expression zum Zelltod führt (Suizidgen), einen damit funktionell verbundenen Promotor P und gegebenenfalls eine Polyadenylierungssequenz;
(c) eine 5'-flankierende Sequenz und/oder eine 3'-flankierende Sequenz, wobei die 5'- und/oder 3'-flankierende Sequenz eine Rekombinase-Targetsequenz enthält.

2. Rekombinanter Vektor nach Anspruch 1, wobei die Transkriptionsfaktorbindestelle den Transkriptionsfaktor p53 bindet.

3. Rekombinanter Vektor nach Anspruch 1 oder 2, wobei die 5'- und/oder die 3'-flankierende Sequenz im wesentlichen aus Rekombinase-Targetsequenzen bestehen.

4. Rekombinanter Vektor nach einem der Ansprüche 1 bis 3, wobei der Vektor ein retroviraler Vektor ist.

5. Rekombinanter Vektor nach Anspruch 4, wobei die 5'- und/oder 3'-flankierenden Sequenzen vollständige oder partielle retrovirale LTR-Bereiche enthalten und mindestens eine Rekombinase-Targetsequenz in den U3- und/oder U5-Bereich des 5'- und/oder 3'-LTR eingebettet ist.

6. Rekombinanter Vektor nach einem der Ansprüche 1 bis 5, wobei die Rekombinase Cre ist.

7. Rekombinanter Vektor nach einem der Ansprüche 1 bis 5, wobei die Rekombinase Flp ist.

8. Rekombinanter Vektor nach Anspruch 6, wobei die Rekombinase-Targetsequenz loxP ist.

9. Rekombinanter Vektor nach Anspruch 7, wobei die Rekombinase-Targetsequenz FRT ist.

10. Rekombinanter Vektor nach einem der Ansprüche 1 bis 9, wobei der Minimalpromotor MP ΔCMV oder ΔMMTV ist.

11. Rekombinanter Vektor nach einem der Ansprüche 1 bis 10, wobei das Suizidgen ein Thymidinkinasegen ist.

12. Rekombinanter Vektor nach einem der Ansprüche 1 bis 10, wobei das Suizidgen für eine Cytosindesaminase kodiert.

13. Rekombinanter Vektor nach einem der Ansprüche 1 bis 12, wobei der Promotor P der MoMULV-LTR-Promotor ist.

14. Rekombinanter Vektor nach einem der Ansprüche 1 bis 13, wobei der Vektor mindestens eine Selektionsmarkerkassette enthält.

15. Rekombinanter Vektor nach der Anspruch 14, wobei die Selektionsmarkerkassette SV40Puro ist.

16. Rekombinanter Vektor nach einem der Ansprüche 1 bis 15, wobei das Suizidgen (i) ein Suizidprotein kodiert, (ii) teilweise oder vollständig komplementär zu einem essentiellen zellulären Gen ist oder (iii) ein cytopathogenes Virus kodiert.

17. Pharmazeutische Zusammensetzung, enthaltend den Vektor nach einem der Ansprüche 1 bis 14 oder 16.

18. Der Vektor nach einem des Ansprüche 1 bis 14 oder 16 zur Verwendung als Arzneimittel.

19. Transduzierte eukaryontische Zelle, erhältlich durch Infizieren einer Tumorzelle, die einen defizienten Transkriptionsfaktor aufweist, mit einem rekombinanten Vektor nach einem der Ansprüche 1 bis 14 oder 16.

20. Verwendung eines Vektores nach einem der Ansprüche 1 bis 14 oder 16 zur Herstellung eines Arzneimittels für die Krebstherapie.

## Claims

1. A recombinant vector comprising:
(a) at least one first transcription cassette containing a sequence coding for a recombinase, a minimal promoter MP functionally linked thereto, a transcription factor binding site and optionally a polyadenylation sequence, wherein the minimal promoter MP depends on the activation by one or several transcription factors;
(b) at least one second transcription cassette containing an nucleic acid sequence which leads to cell death by transcription and optionally expression (suicide gene), a promoter P functionally linked thereto and optionally a polyadenylation sequence;
(c) a 5' flanking sequence and/or a 3' flanking sequence, wherein the 5' and/or 3' flanking sequence contains a recombinase target sequence.

2. The recombinant vector according to claim 1, wherein the transcription factor binding site binds the transcription factor p53.

3. The recombinant vector according to claim 1 or 2, wherein the 5' and/or 3' flanking sequence consists essentially of recombinase target sequences.

4. The recombinant vector according to any of claims 1 to 3, wherein the vector is a retroviral vector.

5. The recombinant vector according to claim 4, wherein the 5' and/or 3' flanking sequences contain complete or partial retroviral LTR regions, and at least one recombinase target sequence is embedded in the U3 and/or U5 region of the 5' and/or 3' LTR.

6. The recombinant vector according to any of claims 1 to 5, wherein the recombinase is Cre.

7. The recombinant vector according to any of claims 1 to 5, wherein the recombinase is Flp.

8. The recombinant vector according to claim 6, wherein the recombinase target sequence is loxP.

9. The recombinant vector according to claim 7, wherein the recombinase target sequence is FRT.

10. The recombinant vector according to any of claims 1 to 9, wherein the minimal promoter MP is ΔCMV or ΔMMTV.

11. The recombinant vector according to any of claims 1 to 10, wherein the suicide gene is a thymidine kinase gene.

12. The recombinant vector according to any of claims 1 to 10, wherein the suicide gene codes for a cytosine deaminase.

13. The recombinant vector according to any of claims 1 to 12, wherein the promoter P is the MoMULV LTR promoter.

14. The recombinant vector according to any of claims 1 to 13, wherein the vector contains at least one selection marker cassette.

15. The recombinant vector according to claim 14, wherein the selection marker cassette is SV40Puro.

16. The recombinant vector according to any of claims 1 to 15, wherein the suicide gene (i) codes for a suicide protein, (ii) is partly or completely complementary to an essential cellular gene, or (iii) codes for a cytopathogenic virus.

17. A pharmaceutical composition containing the vector according to any of claims 1 to 14 or 16.

18. The vector according to any of claims 1 to 14 or 16 for use as a medicament.

19. Transduced eukaryotic cell, obtainable by infecting a tumor cell which has a deficient transcription factor with a recombinant vector according to any of claims 1 to 14 or 16.

20. Use of a vector according to any of claims 1 to 14 or 16 for preparing a medicament for cancer therapy.

## Revendications

1. Vecteur recombinant, comprenant :
(a) au moins une première cassette de transcription, contenant une séquence codant pour une recombinase, un promoteur minimal MP, lui étant lié fonctionnellement, un point de liaison de facteur de transcription et, le cas échéant, une séquence de polyadénylisation, le promoteur minimal MP dépendant de l'excitation par un plusieurs facteurs de transcription ;
(b) au moins une deuxième cassette de transcription, contenant une séquence d'acide nucléique qui mène à la mort cellulaire par transcription et, le cas échéant, expression (gène suicide), un promoteur P lui étant lié fonctionnellement et, le cas échéant, une séquence de polyadénylisation ;
(c) une séquence flanquante 5' et/ou une séquence flanquante 3', sachant que la séquence flanquante 5' et/ou 3' contien(nen)t une séquence cible de recombinase.

2. Vecteur recombinant selon la revendication 1, le point de liaison de facteur de transcription liant le facteur de transcription p53.

3. Vecteur recombinant selon la revendication 1 ou 2, la séquence flanquante 5' et/ou 3' étant essentiellement formée de séquences cibles de recombinase.

4. Vecteur recombinant selon l'une des revendications 1 à 3, le vecteur étant un vecteur rétro-viral.

5. Vecteur recombinant selon la revendication 4, les séquences flanquantes 5' et/ou 3' contenant des zones LTR rétrovirales complètes ou partielles, et au moins une séquence cible de recombinase est incorporée dans la zone U3 et/ou U5 du LTR 5' et/ou 3'.

6. Vecteur recombinant selon l'une des revendications 1 à 5, la recombinase étant Cre.

7. Vecteur recombinant selon l'une des revendications 1 à 5, la recombinase étant Fip.

8. Vecteur recombinant selon la revendication 6, la séquence cible de recombinase étant IoxP.

9. Vecteur recombinant selon la revendication 7, la séquence cible de recombinase étant FRT.

10. Vecteur recombinant selon l'une des revendications 1 à 9, le promoteur minimal MP étant ΔCMV ou ΔMMTV.

11. Vecteur recombinant selon l'une des revendications 1 à 10, le gène suicide étant un gène thymidinekinase.

12. Vecteur recombinant selon l'une des revendications 1 à 10, le gène suicide codant pour une cytosinedésaminase.

13. Vecteur recombinant selon l'une des revendications 1 à 12, le promoteur P étant le promoteur MoMULV-LTR.

14. Vecteur recombinant selon l'une des revendications 1 à 13, le vecteur contenant au moins une cassette de marqueur de sélection.

15. Vecteur recombinant selon la revendication 14, la cassette de marqueur de sélection étant SV40Puro.

16. Vecteur recombinant selon l'une des revendications 1 à 15, le gène suicide (i) codant une protéine suicide, (ii) étant partiellement ou complètement complémentaire d'un gène cellulaire essentiel, ou (iii) codant un virus cytopathogène.

17. Composition pharmaceutique contenant le vecteur selon l'une des revendications 1 à 14 ou 16.

18. Le vecteur selon l'une des revendications 1 à 14 ou 16 pour utilisation en tant que médicament.

19. Cellule eucaryote transduite, obtenue par infection d'une cellule tumorale, présentant un facteur de transcription déficient, avec un vecteur recombinant selon l'une des revendications 1 à 14, ou 16

20. utilisation d'un vecteur selon l'une des revendications 1 à 14 ou 16, pour la préparation d'un médicament pour la carcino-thérapie.
